# FASCICULE DE BREVET EUROPEEN

(11) **EP 1 343 777 B1**
(45) Date de publication et mention de la délivrance du brevet: **06.05.2004**
(21) Numéro de dépôt: 01270534.9
(22) Date de dépôt: 10.12.2001
(51) Int. Cl.: C07D 307/79, C07D 307/80

(54) **DERIVE DE METHANESULFONAMIDO-BENZOFURANE, SON PROCEDE DE PREPARATION ET SON UTILISATION COMME INTERMEDIAIRE DE SYNTHESE**
METHANSUFONAMIDO-BENZOFURAN, HERSTELLUNGSVERFAHREN UND DESSEN VERWENDUNG ALS SYNTHESEZWISCHENPRODUKT
METHANESULPHONAMIDO-BENZOFURAN, PREPARATION METHOD AND USE THEREOF AS SYNTHESIS INTERMEDIATE

(30) Priorité: 11.12.2000 FR 0016070
(43) Date de publication de la demande: 17.09.2003
(73) Titulaire: SANOFI-SYNTHELABO, 75013 Paris (FR)
(72) Inventeur: FINO, Noel, F-04160 Château-Arnoux (FR); LEROY, Corinne, F-05700 Orpierre (FR)
(74) Mandataire: Weber, Mathieu
(86) Numéro de dépôt international: PCT/FR2001/003899
(87) Numéro de publication internationale: WO 2002/048132

(56) Documents cités:
- EP-A- 0 471 609
- WO-A-96/05190

## Description

La présente invention se rapporte, d'une manière générale, à un dérivé de méthanesulfonamido-benzofurane, à son procédé de préparation ainsi qu'à son utilisation comme intermédiaire de synthèse.

Plus précisément, l'invention a pour objet le 2-butyl-5-méthanesulfonamido-benzofurane de formule : 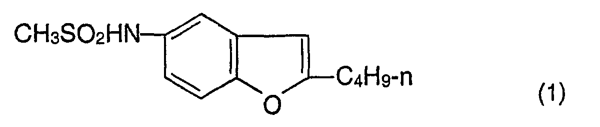 Ce composé s'est révélé particulièrement utile comme produit intermédiaire pour la préparation finale de dérivés aminoalkoxybenzoyl-benzofurane en particulier du 2-butyl-3-(4-[3-(dibutylamino)propoxy]benzoyl)-5-méthanesulfonamido-benzofurane communément appelé dronédarone ainsi que de ses sels pharmaceutiquement acceptables.

Ce dérivé de méthanesulfonamido-benzofurane ainsi que ses sels pharmaceutiquement acceptables ont été décrits dans le brevet EP0471609 de même que ses applications thérapeutiques. Dans le domaine cardiovasculaire, ce composé s'est montré particulièrement intéressant notamment comme agent antiarythmique.

On a rapporté dans le brevet EP0471609, cité précédemment, un procédé pour la préparation de dérivés 3-[4-(aminoalkoxy)benzoyl]benzofurane ou benzo [b] thiophène par fixation d'une chaîne aminoalkoxybenzoyle sur un dérivé de benzofurane ou de benzo [b] thiophène, procédé selon lequel on ajoute d'abord sur le dérivé de benzofurane ou de benzo [b] thiophène en question, un groupement benzoyle comportant en position para un oxygène protégé par un groupement méthyle, on déprotège pour régénérer la fonction hydroxyle et finalement, on introduit la chaîne aminoalkyle souhaitée.
Plus spécifiquement, ce procédé appliqué à la préparation de la dronédarone comporte la suite d'étapes ci-après :
a) réaction du 2-butyl-5-nitro-benzofurane avec le chlorure d'anisoyle en présence de tétrachlorure d'étain selon les conditions de la réaction de Friedel-Crafts et hydrolyse pour former le 2-butyl-3-(4-méthoxy-benzoyl)-5-nitro-benzofurane,
b) déméthylation du composé ainsi obtenu en présence de 2,25 équivalents molaires de chlorure d'aluminium et hydrolyse pour former le 2-butyl-3-(4-hydroxy-benzoyl)-5-nitro-benzofurane,
c) condensation du composé obtenu avec le 1-chloro-3-dibutylamino-propane en présence de carbonate de potassium, pour donner le 2-butyl-3-(4-[3-(dibutylamino)propoxy]benzoyl)-5-nitro-benzofurane,
d) hydrogénation du composé ainsi formé, en présence d'oxyde de platine, ce qui fournit le 5-amino-2-butyl-3-(4-[3-dibutylamino)propoxy]benzoyl-benzofurane,
e) réaction du dérivé de 5-aminobenzofurane ainsi obtenu avec le chlorure de méthanesulfonyle en présence de triéthylamine, ce qui fournit la dronédarone.

Toutefois, ce procédé n'est pas sans présenter certains inconvénients en raison notamment de l'utilisation du chlorure d'aluminium. En effet, la mise en oeuvre de ce procédé à l'échelle industrielle provoque d'importants rejets d'hydroxyde d'aluminium dont le traitement, en vue d'éviter des problèmes de pollution, s'avère onéreux. En outre, l'usage du 2-butyl-3-(4-méthoxy-benzoyl)-5-nitro-benzofurane devrait être évité dans la mesure du possible en raison de ses propriétés mutagènes.

D'autre part, le composé désiré est produit avec un rendement maximum de 60 % à partir du 2-butyl-5-nitro-benzofurane selon ce procédé comportant un nombre relativement élevé d'étapes puisqu'au moins cinq étapes sont nécessaires pour la formation finale de la dronédarone.

La recherche d'un procédé industriel pour la préparation de la dronédarone ou de ses sels pharmaceutiquement acceptables mettant en oeuvre des intermédiaires de synthèse d'accès facile et peu onéreux, selon un procédé plus direct que le procédé antérieur et n'utilisant pas le chlorure d'aluminium reste, par conséquent, d'un intérêt incontestable.

On a rapporté dans J.Med.Chem. 1984, 27, 1057-1066 une méthode plus convergente pour fixer une chaîne aminoalkoxybenzoyle sur un dérivé de benzo [b] thiophène sans transiter par une étape de protection/déprotection de la fonction hydroxyle. Toutefois, ce procédé propose encore à la page 1064 l'utilisation du chlorure d'aluminium en quantités particulièrement importantes puisque de l'ordre de 9 équivalents molaires.

Selon cette méthode, on condense dans une phase organique constituée de dichloréthane le dérivé de benzo [b] thiophène en question avec le chlorhydrate du chlorure du dérivé aminoalkoxybenzoyle et ce, en présence de chlorure d'aluminium.

Après hydrolyse, le chlorhydrate du dérivé 3-[4-(aminoalkoxy)benzoyl] benzo [b] thiophène désiré est récupéré en partie de la phase organique et en partie de la phase aqueuse par trois extractions au chloroforme puis traité par l'hydroxyde de sodium.

Dans le cadre de l'élaboration de la présente invention, on a appliqué ce procédé au départ du 2-butyl-5-nitro-benzofurane en vue de préparer directement le 2-butyl-3-(4-[3-(dibutylamino)propoxy]benzoyl)-5-nitro-benzofurane et ce, par mise en oeuvre des étapes suivantes :
- traitement du 2-butyl-5-nitro-benzofurane au moyen du chlorhydrate du chlorure de 4-[3-(dibutylamino)propoxy]benzoyle en présence de 9 équivalents molaires de chlorure d'aluminium et ce, dans une phase organique,
- hydrolyse, récupération du chlorhydrate de 2-butyl-3-(4-[3-(dibutylamino)propoxy]benzoyl)-5-nitro-benzofurane et traitement par l'hydroxyde de sodium pour former le 2-butyl-3-(4-[3-(dibutylamino)propoxy]benzoyl)-5-nitro-benzofurane souhaité.

Cependant, ce procédé s'est avéré inadapté au niveau industriel en raison d'une part de l'énorme quantité d'hydroxyde d'aluminium ainsi produit et d'autre part du taux important d'impuretés récoltées et, par conséquent, du faible rendement fourni en 2-butyl-3-(4-[3-(dibutylamino)propoxy]-5-nitro-benzofurane (20 à 30 %).

Or, on a trouvé de manière surprenante, qu'il est possible, à partir du 2-butyl-5-méthanesulfonamido-benzofurane et en utilisant des quantités appropriées d'un acide de Lewis dans une réaction de Friedel-Crafts, d'obtenir directement le chlorhydrate de dronédarone avec d'excellents rendements puisque d'au moins 85 %, ce chlorhydrate pouvant être récupéré de manière remarquablement avantageuse puisqu'on le retrouve en quasi totalité non pas dans la phase aqueuse comme on aurait pu le prévoir mais dans la phase organique utilisée, ce qui évite la nécessité de procéder à plusieurs extractions de cette même phase aqueuse comme dans le procédé antérieur.

En outre, le 2-butyl-5-méthanesulfonamido-benzofurane peut être obtenu lui-même avec grande facilité et rendements importants puisque supérieurs à 75 % à partir du 5-amino-2-butyl-benzofurane et même à partir du précurseur de celui-ci à savoir le 2-butyl-5-nitro-benzofurane.

Le 2-butyl-5-méthanesulfonamido-benzofurane est un produit nouveau qui s'obtient facilement sous forme cristalline au contraire du 2-butyl-5-nitro-benzofurane dont l'état cristallin peut être difficile à atteindre. Ce dérivé méthanesulfonamido présente donc un avantage indéniable sur le dérivé nitro en question.

En conséquence, l'invention se rapporte au 2-butyl-5-méthanesulfonamido-benzofurane en tant que produit industriel nouveau utile notamment comme intermédiaire de synthèse, par exemple pour la préparation de la dronédarone ou de ses sels pharmaceutiquement acceptables.

Ainsi, selon l'invention, on prépare le 2-butyl-5-méthanesulfonamido-benzofurane en faisant réagir le 5-amino-2-butyl-benzofurane avec le chlorure de méthanesulfonyle ou l'anhydride méthanesulfonique, la réaction ayant lieu en présence d'un accepteur d'acide tel que la triéthylamine ou l'ammoniac, ce qui fournit le composé désiré.
Généralement, la réaction se déroule à la température ambiante et dans un ou plusieurs solvants apolaires de préférence choisis parmi des hydrocarbures halogénés et des éthers tels que par exemple le méthyltertbutyléther, le tétrahydrofurane, le dichlorométhane ou le dichloréthane.

Le 5-amino-2-butyl-benzofurane, quant à lui peut être préparé en hydrogénant le 2-butyl-5-nitro-benzofurane en présence d'un catalyseur approprié, ce qui fournit le composé souhaité.

Comme catalyseur, on utilise habituellement un dérivé de platine tel que l'oxyde de platine ou un système formiate d'ammonium/charbon palladié, l'hydrogénation ayant lieu à température ambiante et éventuellement sous pression par exemple sous une pression de l'ordre de 20 à 30 bars.

Cette hydrogénation, qui s'accomplit avec des rendements excellents atteignant jusqu'à 100 %, présente des avantages incontestables par rapport à l'hydrogénation du 2-butyl-3-(4-[3-(dibutylamino)propoxy]benzoyl)-5-nitro-benzofurane du procédé antérieur. En effet, le 2-butyl-5-nitro-benzofurane, hormis le groupement nitro, ne comporte aucun autre groupement fonctionnel susceptible d'être modifié par cette réaction contrairement au dérivé 2-butyl-3-(4-[3-(dibutylamino)propoxy]benzoyl)-5-nitro-benzofurane dont l'hydrogénation peut s'avérer difficile en raison des produits secondaires formés.

Comme indiqué précédemment, le 2-butyl-5-méthanesulfonamido-benzofurane peut être utilisé pour la préparation de la dronédarone.

Ainsi, selon l'invention, on prépare la dronédarone en formant d'abord son chlorhydrate, c'est-à-dire en faisant réagir, dans une phase organique, le 2-butyl-5-méthanesulfonamido-benzofurane avec le chlorhydrate du chlorure de 4-[3-(dibutylamino)propoxy]benzoyle et ce, en présence d'un acide de Lewis comme catalyseur et, en hydrolysant pour former le chlorhydrate de dronédarone que l'on récupère dans la phase organique.

La réaction, entreprise dans les conditions de la réaction de Friedel-Crafts, est effectuée habituellement à la température ambiante et dans une phase organique constituée d'un ou de plusieurs solvants choisis parmi des hydrocarbures halogénés ou non, de préférence de type aliphatique, alicyclique ou aromatique. Généralement, on utilise des hydrocarbures halogénés, de préférence chlorés, de type aliphatique, alicyclique ou aromatique tels que par exemple le dichlorométhane, le dichloréthane ou le chlorobenzène.

En outre, l'acide de Lewis peut être le chlorure d'aluminium, le chlorure de zinc, le trifluorure de bore, le chlorure stannique, le tétrachlorure de titane ou, de préférence le chlorure ferrique. On peut aussi utiliser un mélange de ces acides de Lewis. Cet acide de Lewis est utilisé à des concentrations n'excédant pas 5 équivalents molaires, en particulier à raison de 2 à 5 équivalents molaires. Plus particulièrement , cet acide de Lewis est utilisé à des concentrations n'excédant pas 4 équivalents molaires. Plus particulièrement encore, cet acide de Lewis est utilisé à des concentrations n'excédant pas 3 équivalents molaires en particulier à raison de 2 à 3 équivalents molaires, de préférence 2,5 équivalents molaires.

Enfin, le chlorhydrate du chlorure de 4-[3-(dibutylamino)propoxy]benzoyle est mis en oeuvre à des concentrations de l'ordre de 1 à 1,3 équivalent molaire.

Le chlorhydrate de dronédarone ainsi obtenu est alors transformé après isolement en dronédarone par traitement au moyen d'un agent basique tel qu'un hydroxyde de métal alcalin par exemple l'hydroxyde de sodium, un carbonate de métal alcalin ou un hydrogénocarbonate de métal alcalin tel que l'hydrogénocarbonate de sodium, ce qui fournit le composé désiré.

Selon une mise en oeuvre préférée de l'invention, on prépare la dronédarone sans isolement de son chlorhydrate formé transitoirement, c'est-à-dire dans le milieu même où ce chlorhydrate est préparé.

En conséquence, selon une variante de l'invention, on prépare la dronédarone moyennant un procédé selon lequel, dans une phase organique constituée d'un ou de plusieurs solvants choisis parmi des hydrocarbures halogénés ou non, on fait réagir le 2-butyl-5-méthanesulfonamido-benzofurane avec le chlorhydrate du chlorure de 4-[3-(dibutylamino)propoxy]benzoyle et ce, en présence d'un maximum de 5 équivalents molaires, en particulier d'un maximum de 4 équivalents molaires et de préférence d'un maximum de 3 équivalents molaires d'un acide de Lewis comme catalyseur, on hydrolyse pour obtenir transitoirement et sans isolement le chlorhydrate de dronédarone que l'on récupère dans la phase organique et on traite le chlorhydrate formé au moyen d'un agent basique, ce qui fournit la dronédarone.

Selon un autre aspect de l'invention, la dronédarone peut être obtenue par mise en oeuvre d'un procédé en trois étapes au départ du 2-butyl-5-nitro-benzofurane.

En conséquence, un autre objet de l'invention se rapporte à la préparation de la dronédarone au départ du 2-butyl-5-nitro-benzofurane selon un procédé par lequel :
a) on hydrogène le 2-butyl-5-nitro-benzofurane en présence d'un catalyseur approprié, pour former le 5-amino-2-butyl-benzofurane,
b) on fait réagir le composé ainsi obtenu avec le chlorure de méthanesulfonyle ou l'anhydride méthanesulfonique, la réaction ayant lieu en présence d'un accepteur d'acide, pour former le 2-butyl-5-méthanesulfonamido-benzofurane,
c) on fait réagir, dans une phase organique constituée d'un ou de plusieurs solvants choisis parmi des hydrocarbures halogénés ou non, le dérivé méthanesulfonamido ainsi obtenu, avec le chlorhydrate du chlorure de 4-[3-(dibutylamino)propoxy]benzoyle et ce, en présence d'un maximum de 5 équivalents molaires, en particulier d'un maximum de 4 équivalents molaires et de préférence d'un maximum de 3 équivalents molaires d'un acide de Lewis comme catalyseur, on hydrolyse pour obtenir transitoirement et sans isolement le chlorhydrate de dronédarone que l'on récupère dans la phase organique et on traite le chlorhydrate formé au moyen d'un agent basique, ce qui fournit la dronédarone.

Par la suite, la dronédarone obtenue selon l'une ou l'autre méthode ou variante de l'invention peut être traitée, si nécessaire, avec un acide organique ou inorganique pour former un sel pharmaceutiquement acceptable de ce composé.

Le chlorhydrate du chlorure de 4-[3-(dibutylamino)propoxy]benzoyle, quant à lui, peut être préparé selon la suite d'étapes ci-après :
a) on fait réagir le 1-dibutylamino-3-chloro-propane avec un p-hydroxybenzoate d'alkyle en C₁-C₄ par exemple le p-hydroxybenzoate de méthyle et ce, en présence d'un agent basique tel qu'un carbonate de métal alcalin par exemple le carbonate de potassium pour obtenir un 4-[3-(dibutylamino)propoxy]benzoate de méthyle,
b) on saponifie l'ester ainsi obtenu, en présence d'un hydroxyde de métal alcalin par exemple l'hydroxyde de sodium puis on traite le sel ainsi formé au moyen d'acide chlorhydrique pour donner le chlorhydrate de l'acide 4-[3-(dibutylamino)propoxy]benzoïque,
c) on traite alors le chlorhydrate ainsi formé au moyen d'un agent de chloration par exemple le chlorure de thionyle, pour obtenir le composé désiré.

Cette mise en oeuvre du procédé de l'invention pour la préparation de la dronédarone s'est avérée supérieure au procédé du brevet EP0471609 en raison notamment d'un nombre réduit d'étapes à savoir trois au lieu de cinq au départ du 2-butyl-5-nitro-benzofurane et d'un rendement global plus important puisqu'il est supérieur à 65 % voire à 70 %.

D'autre part, ce procédé de l'invention fait appel, à chaque étape, à des intermédiaires benzofuraniques de structure relativement simples, par conséquent peu onéreux, au contraire du procédé antérieur qui met en oeuvre à chacune de ses étapes des dérivés de benzofurane de structure assez élaborée.

Les exemples non limitatifs suivants illustrent l'invention.

### PREPARATIONS

### A. 1-Dibutylamino-3-chloro-propane

Dans un réacteur de 1 I, on introduit 288,4 g (3,392 moles) d'ammoniaque à 20% puis on ajoute, en 10 minutes et à température ambiante (22 ± 2°C), 618 g (1,696 mole) de chlorhydrate de 1-dibutylamino-3-chloro-propane (titre 66,5 %). On agite durant 45 minutes à température ambiante et on laisse décanter pendant 30 minutes. On élimine la phase aqueuse inférieure (pH=11 ) et, avec 300 ml d'eau désionisée, on effectue à température ambiante un lavage de la phase organique. On agite durant 30 minutes, on décante pendant 30 minutes et on soutire la phase aqueuse inférieure (pH=9).

De cette manière, on recueille 346,3 g de composé souhaité.
Rendement : 99,4 %

### B. 4-[3-(Dibutylamino)propoxy]benzoate de méthyle

Dans un ballon de 2 l, on introduit 200 g (1,3 mole) de p-hydroxybenzoate de méthyle et 1,6 l de N,N-diméthylformamide. On agite le mélange et on y ajoute 232g (1,66 mole) de carbonate de potassium. On chauffe à 100°C puis on introduit, en 10 minutes, le 1-dibutylamino-3-chloro-propane préparé à l'étape **A**. précédente. On maintient le milieu réactionnel durant 1 heure à 100 ± 2°C puis on refroidit à 25°C. On filtre les sels minéraux, on rince avec 2 fois 50 ml de N,N-diméthylformamide et on concentre le filtrat à l'évaporateur rotatif jusqu'à la température de 85°C et la pression de 5 mmHg.

De cette manière, on obtient 472,7 g de produit désiré sous forme d'une huile orangée.

### Pureté (CLHP ou chromatographie liquide haute pression)

Composé désiré : 99,7 %
p-Hydroxybenzoate de méthyle: 0,1 %

### C. Chlorhydrate de l'acide 4-[3-(dibutylamino)propoxy]benzoïque

Dans un ballon de 2 I, on introduit 436,3 g de 4-[3-(dibutylamino)propoxy]benzoate de méthyle et 1,092 I de méthanol. On agite le mélange et on introduit, en environ 5 minutes, 360 g (1,8 mole) d'hydroxyde de sodium à 20 %.
On chauffe à 65°C durant environ 30 minutes et on maintient à cette température pendant 2 heures. On refroidit le milieu réactionnel à 30°C et on concentre à l'évaporateur rotatif (température du bain : 30°C, pression 30 mmHg), ce qui fournit 937 g de résidu que l'on dilue par ajout de 2,8 I d'eau désionisée. On refroidit la solution à 10 ± 2°C puis, sans dépasser 20°C, on introduit 260 ml (environ 3 moles) d'acide chlorhydrique à 36 %.
On vérifie que le pH est inférieur à 1 puis on refroidit la suspension à 10 ± 2°C. On maintient cette température pendant 30 minutes, on essore les cristaux formés et on lave le gâteau avec 2 fois 200 ml d'eau désionisée. On sèche ensuite en étuve ventilée à 50°C jusqu'à poids constant (24 heures).

De cette manière, on obtient 416,2 g de composé désiré.
Rendement: 100 %.

### Pureté (CLHP)

Composé désiré : 99,5 %
4-[3-(Dibutylamino)propoxy]benzoate de méthyle : 0,1 %

### D. Chlorhydrate du chlorure de 4-[3-(dibutylamino) propoxy]benzoyle

Dans un ballon, on introduit 63,3 g (0,184 mole) de chlorhydrate de l'acide 4-[3-(dibutylamino)propoxy]benzoïque, 300 ml de chlorobenzène et 2 gouttes de N,N-diméthylformamide. Tout en maintenant le mélange sous atmosphère inerte, on introduit, en environ 45 minutes, 43,8 g (0,368 mole) de chlorure de thionyle. On maintient durant 1 heure à 85 ± 1°C puis on distille sous vide progressif environ 115 g d'un mélange de chlorobenzène et de chlorure de thionyle.

De cette manière, on obtient le composé désiré sous forme d'une huile et sous forme brute.

### EXEMPLE 1

### 5-Amino-2-butyl-benzofurane

### Méthode 1

Dans un appareil à hydrogéner, on introduit 182 g de 2-butyl-5-nitro-benzofurane et 730 ml d'éthanol puis on ajoute 9,1 g d'oxyde de platine à 97% environ. On fait barboter un courant d'hydrogène tout en agitant sous une pression de 25 bars (la température s'élève spontanément à 60°C) et on refroidit à 20°C. On filtre, rince par 400 ml d'éthanol et on concentre à 50°C sous vide.

De cette manière, on obtient le 5-amino-2-butyl-benzofurane.
Rendement pondéral : 99,2 %

### Méthode II

Dans un réacteur clos, on introduit 1 g de 2-butyl-5-nitro-benzofurane et 4 ml d'éthanol puis on ajoute 1,48 g de formiate d'ammonium et 0,1 g de charbon palladié à 5 %. On agite et on chauffe à 50°C pendant 5 heures puis on refroidit à 20°C et on filtre. On rince par 4 ml d'éthanol et on concentre à 50°C, sous vide.

De cette manière, on obtient le 5-amino-2-butyl-benzofurane.

### EXEMPLE 2

### 2-Butyl-5-méthanesulfonamido-benzofurane

Dans un ballon, on introduit 100 g de 5-amino-2-butyl-benzofurane, 192 ml de tétrahydrofurane et 96 ml de méthyltertbutyléther. On ajoute, à 20°C, 59,35 g de chlorure de méthanesulfonyle puis 43,23 g d'ammoniaque à 20 %. On introduit ensuite 44,51 g de chlorure de méthanesulfonyle puis 86,47 g d'ammoniaque à 20 %. On ajoute alors 48 ml d'eau, on décante et on lave la phase organique deux fois avec 211 g de solution aqueuse à 10 % de chlorure de sodium. On décante et on concentre à 40°C, sous vide pour obtenir le composé désiré sous forme brute (rendement pondéral : 100 %).
On introduit 135,6 g de résidu ainsi obtenu, dans 1220 ml de méthyltertbutyléther et on ajoute 13,56 g de noir de carbone.
On agite au reflux pendant 15 minutes, filtre et rince avec 204 ml de méthyltertbutyléther. On chauffe au reflux le filtrat puis on refroidit à 40°C. On ensemence alors le milieu avec du 2-butyl-3-méthanesulfonamido-benzofurane, on refroidit à - 5°C et on agite à cette température durant 30 minutes. On filtre et rince à - 5°C par deux fois 1 volume de méthyltertbutyléther.

De cette manière, on obtient le composé désiré avec un rendement pondéral de 78,5 %.
Spectre R.M.N. (résonance magnétique nucléaire) (300 MHz)
Solvant : CDCl₃
Concentration : 40 mg/ml
Température d'analyse : 300 K

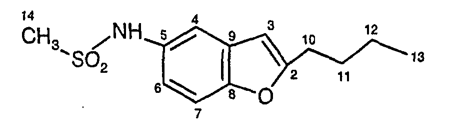

| **Placements chimiques δ ± 0,01 ppm** | **Multiplicité** | **Intégration** | **Constantes de couplage \|J\| ± 0,5 Hz** | **Attribution** |
|---|---|---|---|---|
| 7,42 | Doublet | 1 | ⁴J_{H-H}≈ 2,0 | H(4) |
| 7,36 | Doublet | 1 | ³J_{H-H}≈ 8,5 | H(7) |
| 7,08 | Doublet de doublets | 1 | ³J_{H-H}≈ 8,5 ⁴J_{H-H}≈ 2,0 | H(6) |
| 6,83-6,89 | Signaux larges | 1 | - | NH |
| 6,35 | Singulet | 1 | - | H(3) |
| 2,97 | Singulet | 3 | - | CH₃SO₂ |
| 2,76 | Triplet | 2 | ³J_{H-H}≈ 7,5 | CH₂(10) |
| 1,72 | Quintuplet | 2 | ³J_{H-H}≈ 7,5 | CH₂(11) |
| 1,42 | Sextuplet | 2 | ³J_{H-H}≈ 7,5 | CH₂(12) |
| 0,95 | Triplet | 3 | ³J_{H-H}≈ 7,5 | CH₃(13) |

### EXEMPLE 3

### Chlorhydrate de dronédarone

On constitue d'abord un mélange de 8 g de 2-butyl-5-méthanesulfonamido-benzofurane, 11,9 g de chlorhydrate du chlorure de 4-[3-(dibutylamino)propoxy]benzoyle et de 24 ml de dichlorométhane. On introduit ensuite ce mélange dans un milieu formé de 11,9 g de chlorure ferrique et de 24 ml de dichlorométhane. On porte le milieu réactionnel au reflux pendant 2 heures et on refroidit à 20°C. On ajoute 40 ml d'eau et on décante.

De cette manière, on obtient le chlorhydrate de dronédarone sous forme brute. Rendement chimique : 90 %.

### EXEMPLE 4

### Chlorhydrate de dronédarone

### A. Dronédarone

On lave deux fois le chlorhydrate de dronédarone obtenu à l'Exemple 3 précédent et ce, avec 24 ml d'eau, puis avec 16 ml d'une solution aqueuse à 10 % d'hydroxyde de sodium et enfin avec 16 ml d'eau.

De cette manière, on obtient la dronédarone sous forme brute.
Rendement chimique : 86 % .

### B. Chlorhydrate de dronédarone

On concentre la dronédarone sous forme brute obtenue au paragraphe **A.** précédent et on reprend dans 24 ml d'isopropanol. On ajoute 3,24 g d'acide chlorhydrique à 37 % puis on refroidit, à 0°C. On filtre et on lave avec 8 ml d'isopropanol.

De cette manière, on obtient le chlorhydrate de dronédarone.

### EXEMPLE 5

### Chlorhydrate de dronédarone - Préparation à l'échelle pilote

### 5.1 Chlorhydrate de dronédarone

On constitue d'abord un mélange de 10 kg de 2-butyl-5-méthanesulfonamide benzofurane, 13 kg de chlorhydrate de 4-[3-(dibutylamino)propoxy]benzoyle et de 70 I de dichlorométhane. On introduit ensuite sur ce mélange 19.9 kg de chlorure d'aluminium (on utilise ainsi 4 équivalents molaires d'acide de Lewis; on a utilisé 2,5 équivalents molaires de chlorure ferrique pour l'exemple 3 précédent). On agite le milieu à 25°C pendant deux heures. On ajoute ce milieu sur 60 I d'eau et on décante.

De cette manière on obtient le chlorhydrate de dronédarone sous forme brute.
Rendement chimique = 90%.

### 5.2 Dronédarone puis chlorhydrate de dronédarone

On lave 5 fois le chlorhydrate de dronédarone obtenu à l'étape précédente, avec 60 I d'eau. On concentre la dronédarone et on reprend avec 94 l d'isopropanol. On ajout 1.8 kg d'eau et 0.38 kg d'acide chlorhydrique. On refroidit à -5°C. On filtre, on lave avec 22 I d'isopropanol.

De cette manière, on obtient le chlorhydrate de la dronédarone.
Rendement chimique = 90%

## Revendications

1. 2-Butyl-5-méthanesulfonamido-benzofurane.

2. Procédé de préparation du 2-butyl-5-méthanesulfonamido-benzofurane, **caractérisé en ce que** l'on fait réagir le 5-amino-2-butyl-benzofurane avec le chlorure de méthanesulfonyle ou l'anhydride méthanesulfonique, la réaction ayant lieu en présence d'un accepteur d'acide, ce qui fournit le composé désiré.

3. Procédé de préparation de la dronédarone (2-butyl-3-(4-[3-(dibutylamino)propoxy]benzoyl)-5-méthanesulfonamido-benzofurane), **caractérisé en ce que**, dans une phase organique constituée d'un ou de plusieurs solvants choisis parmi des hydrocarbures halogénés ou non, on fait réagir le 2-butyl-5-méthanesulfonamido-benzofurane avec le chlorhydrate du chlorure de 4-[3-(dibutylamino)propoxy]benzoyle et ce, en présence d'un maximum de 5 équivalents molaires d'un acide de Lewis comme catalyseur, puis on hydrolyse pour former le chlorhydrate de dronédarone que l'on récupère dans la phase organique, que l'on isole et que l'on traite au moyen d'un agent basique, pour obtenir le composé désiré.

4. Procédé selon la revendication 3, **caractérisé en ce que** l'on fait réagir le 2-butyl-5-méthanesulfonamido-benzofurane avec le chlorhydrate du chlorure de 4-[3-(dibutylamino)propoxy]benzoyle en présence d'un maximum de 4 équivalents molaires de l'acide de Lewis.

5. Procédé selon la revendication 3 ou 4, **caractérisé en ce que** l'on fait réagir le 2-butyl-5-méthanesulfonamido-benzofurane avec le chlorhydrate du chlorure de 4-[3-(dibutylamino)propoxy]benzoyle en présence d'un maximum de 3 équivalents molaires de l'acide de Lewis.

6. Procédé de préparation de la dronédarone, **caractérisé en ce que**, dans une phase organique constituée d'un ou de plusieurs solvants choisis parmi des hydrocarbures halogénés ou non, on fait réagir le 2-butyl-5-méthanesulfonamido-benzofurane avec le chlorhydrate du chlorure de 4-[3-(dibutylamino)propoxy]benzoyle et ce, en présence d'un maximum de 5 équivalents molaires d'un acide de Lewis comme catalyseur puis on hydrolyse pour obtenir transitoirement et sans isolement le chlorhydrate de dronédarone que l'on récupère dans la phase organique et que l'on traite au moyen d'un agent basique, ce qui fournit le composé désiré.

7. Procédé selon la revendication 6, **caractérisé en ce que** l'on fait réagir le 2-butyl-5-méthanesulfonamido-benzofurane avec le chlorhydrate du chlorure de 4-[3-(dibutylamino)propoxy]benzoyle en présence d'un maximum de 4 équivalents molaires de l'acide de Lewis.

8. Procédé selon la revendication 6 ou 7, **caractérisé en ce que** l'on fait réagir le 2-butyl-5-méthanesulfonamido-benzofurane avec le chlorhydrate du chlorure de 4-[3-(dibutylamino)propoxy]benzoyle en présence d'un maximum de 3 équivalents molaires de l'acide de Lewis.

9. Procédé de préparation de la dronédarone, **caractérisé en ce que** :
a) l'on hydrogène le 2-butyl-5-nitro-benzofurane en présence d'un catalyseur approprié, pour former le 5-amino-2-butyl-benzofurane,
b) l'on fait réagir le composé ainsi obtenu avec le chlorure de méthanesulfonyle ou l'anhydride méthanesulfonique, la réaction ayant lieu en présence d'un accepteur d'acide, pour former le 2-butyl-5-méthanesulfonamido-benzofurane,
c) l'on fait réagir, dans une phase organique constituée d'un ou de plusieurs solvants choisis parmi des hydrocarbures halogénés ou non, le dérivé méthanesulfonamido ainsi obtenu, avec le chlorhydrate du chlorure de 4-[3-(dibutylamino)propoxy]benzoyle et ce, en présence d'un maximum de 5 équivalents molaires d'un acide de Lewis comme catalyseur puis on hydrolyse pour obtenir transitoirement et sans isolement le chlorhydrate de dronédarone que l'on récupère dans la phase organique et que l'on traite au moyen d'un agent basique, ce qui fournit le composé désiré.

10. Procédé selon la revendication 9, **caractérisé en ce que**, à l'étape c), on fait réagir le dérivé méthanesulfonamido ainsi obtenu, avec le chlorhydrate du chlorure de 4-[3-(dibutylamino)propoxy]benzoyle, en présence d'un maximum de 4 équivalents molaires de l'acide de Lewis.

11. Procédé selon la revendication 9 ou 10, **caractérisé en ce que**, à l'étape c), on fait réagir le dérivé méthanesulfonamido ainsi obtenu, avec le chlorhydrate du chlorure de 4-[3-(dibutylamino)propoxy]benzoyle, en présence d'un maximum de 3 équivalents molaires de l'acide de Lewis.

12. Procédé selon une des Revendications 3 à 11, **caractérisé en ce que** la phase organique est constituée d'un ou de plusieurs solvants choisis parmi des hydrocarbures halogénés de type aliphatique, alicyclique ou aromatique.

13. Procédé selon une des Revendications 3 à 12, **caractérisé en ce que** l'acide de Lewis est choisi dans le groupe constitué par le chlorure d'aluminium, le chlorure de zinc, le trifluorure de bore, le chlorure stannique, le tétrachlorure de titane, le chlorure ferrique et les mélanges de ceux-ci.

14. Procédé selon une des Revendications 3 à 13, **caractérisé en ce que** l'acide de Lewis est le chlorure ferrique.

15. Procédé selon une des Revendications 3, 6, 9, 13 et 14, **caractérisé en ce que** l'acide de Lewis est utilisé à raison de 2 à 5 équivalents molaires.

16. Procédé selon une des Revendications 3 à 11, 13 et 14, **caractérisé en ce que** l'acide de Lewis est utilisé à raison de 2 à 3 équivalents molaires.

17. Procédé selon une des Revendications 3 à 16, **caractérisé en ce que** le chlorhydrate du chlorure de 4-[3-(dibutylamino)propoxy]benzoyle est utilisé à des concentrations de l'ordre de 1 à 1,3 équivalent molaire.

18. Procédé selon une des Revendications 3 à 17, **caractérisé en ce que** l'agent basique est l'hydroxyde de sodium ou l'hydrogénocarbonate de sodium.

## Patentansprüche

1. 2-Butyl-5-methansulfonamido-benzofuran.

2. Verfahren zur Herstellung von 2-Butyl-5-methansulfonamido-benzofuran, **dadurch gekennzeichnet, daß** man 5-Amino-2-butyl-benzofuran mit Methansulfonylchlorid oder Methansulfonsäureanhydrid in Gegenwart eines Säureakzeptors unter Bildung der gewünschten Verbindung umsetzt.

3. Verfahren zur Herstellung von Dronedaron (2-Butyl-3-(4-[3-(dibutylamino)-propoxy]-benzoyl)-5-methansulfonamido-benzofuran), **dadurch gekennzeichnet, daß** man in einer organischen Phase, die aus einem oder mehreren Lösungsmitteln ausgewählt aus gegebenenfalls halogenierten Kohlenwasserstoffen gebildet ist, 2-Butyl-5-methansulfonamido-benzofuran mit 4-[3-(Dibutylamino)-propoxy]-benzoylchlorid-Hydrochlorid in Gegenwart eines Maximums von 5 Moläquivalenten einer Lewis-Säure als Katalysator umsetzt, dann zur Bildung von Dronedaron-Hydrochlorid hydrolysiert, welches man aus der organischen Phase gewinnt, isoliert und mit einem basischen Mittel behandelt zur Bildung der gewünschten Verbindung.

4. Verfahren nach Anspruch 3, **dadurch gekennzeichnet, daß** man 2-Butyl-5-methansulfonamido-benzofuran mit 4-[3-(Dibutylamino)-propoxy]-benzoylchlorid-Hydrochlorid in Gegenwart eines Maximums von 4 Moläquivalenten der Lewis-Säure umsetzt.

5. Verfahren nach Anspruch 3 oder 4, **dadurch gekennzeichnet, daß** man 2-Butyl-5-methansulfonamido-benzofuran in Gegenwart eines Maximums von 3 Moläquivalenten der Lewis-Säure mit 4-[3-(Dibutylamino)-propoxy]-benzoylchlorid-Hydrochlorid umsetzt.

6. Verfahren zur Herstellung von Dronedaron, **dadurch gekennzeichnet, daß** man in einer organischen Phase, die aus einem oder mehreren Lösungsmitteln ausgewählt aus gegebenenfalls halogenierten Kohlenwasserstoffen gebildet ist, 2-Butyl-5-methansulfonamido-benzofuran mit 4-[3-(Dibutylamino)-propoxy]-benzoylchlorid-Hydrochlorid in Gegenwart eines Maximums von 5 Moläquivalenten einer Lewis-Säure als Katalysator umsetzt, dann zur zwischenzeitlichen Bildung von Dronedaron-Hydrochlorid hydrolysiert, ohne daß dieses isoliert wird, welches man aus der organischen Phase gewinnt und mit einem basischen Mittel zur Bildung der gewünschten Verbindung behandelt.

7. Verfahren nach Anspruch 6, **dadurch gekennzeichnet, daß** man 2-Butyl-5-methansulfonamido-benzofuran mit 4-[3-(Dibutylamino)-propoxyl-benzoylchlorid-Hydrochlorid in Gegenwart eines Maximums von 4 Moläquivalenten der Lewis-Säure umsetzt.

8. Verfahren nach Anspruch 6 oder 7, **dadurch gekennzeichnet, daß** man 2-Butyl-5-methansulfonamid-benzofuran mit 4-[3-(Dibutylamino)-propoxy]-benzoylchlorid-Hydrochlorid in Gegenwart eines Maximums von 3 Moläquivalenten der Lewis-Säure umsetzt.

9. Verfahren zur Herstellung von Dronedaron, **dadurch gekennzeichnet, daß** man:
a) 2-Butyl-5-nitro-benzofuran in Gegenwart eines geeigneten Katalysators zur Bildung von 5-Amino-2-butyl-benzofuran hydriert,
b) die in dieser Weise erhaltene Verbindung mit Methansulfonylchlorid in Gegenwart eines Säureakzeptors zur Bildung von 2-Butyl-5-methansulfonamidobenzofuran umsetzt,
c) das in dieser Weise erhaltene Methansulfonamido-Derivat in einer organischen Phase, die aus einem oder mehreren Lösungsmitteln ausgewählt aus gegebenenfalls halogenierten Kohlenwasserstoffen gebildet ist, mit 4-[3-(Dibutylamino)-propoxy]-benzoylchlorid-Hydrochlorid in Gegenwart eines Maximums von 5 Moläquivalenten einer Lewis-Säure als Katalysator umsetzt und dann hydrolysiert, um zwischenzeitlich und ohne es zu isolieren Dronedaron-Hydrochlorid zu bilden, welches man aus der organischen Phase gewinnt und mit einem basischen Mittel zur Bildung der gewünschten Verbindung behandelt.

10. Verfahren nach Anspruch 9, **dadurch gekennzeichnet, daß** man das in dieser Weise erhaltene Methansulfonamido-Derivat in der Stufe c) in Gegenwart eines Maximums von 4 Moläquivalenten der Lewis-Säure mit 4-[3-(Dibutylamino)-propoxy)-benzoylchlorid-Hydrochlorid umsetzt.

11. Verfahren nach Anspruch 9 oder 10, **dadurch gekennzeichnet, daß** man das in dieser Weise erhaltene Methansulfonamido-Derivat in der Stufe c) in Gegenwart eines Maximums von 3 Moläquivalenten der Lewis-Säure mit 4-[3-(Dibutylamino)-propoxy]-benzoylchlorid-Hydrochlorid umsetzt.

12. Verfahren nach einem der Ansprüche 3 bis 11, **dadurch gekennzeichnet, daß** die organische Phase aus einem oder mehreren Lösungsmitteln ausgewählt aus aliphatischen, alicyclischen oder aromatischen halogenierten Kohlenwasserstoffen gebildet ist.

13. Verfahren nach einem der Ansprüche 3 bis 12, **dadurch gekennzeichnet, daß** die Lewis-Säure aus der Gruppe ausgewählt ist, die aus Aluminiumchlorid, Zinkchlorid, Bortrifluorid, Zinn(IV)-chlorid, Titantetrachlorid, Eisen(III)-chlorid und Mischungen davon besteht.

14. Verfahren nach einem der Ansprüche 3 bis 13, **dadurch gekennzeichnet, daß** die Lewis-Säure Eisen(III)-chlorid ist.

15. Verfahren nach einem der Ansprüche 3, 6, 9, 13 und 14, **dadurch gekennzeichnet, daß** die Lewis-Säure in einer Menge von 2 bis 5 Moläquivalenten verwendet wird.

16. Verfahren nach einem der Ansprüche 3 bis 11, 13 und 14, **dadurch gekennzeichnet, daß** die Lewis-Säure in einer Menge von 2 bis 3 Moläquivalenten verwendet wird.

17. Verfahren nach einem der Ansprüche 3 bis 16, **dadurch gekennzeichnet, daß** 4-[3-(Dibutylamino)-propoxy]-benzoylchlorid-Hydrochlorid in einer Konzentration im Bereich von 1 bis 1,3 Moläquivalenten verwendet wird.

18. Verfahren nach einem der Ansprüche 3 bis 17, **dadurch gekennzeichnet, daß** das basische Mittel Natriumhydroxid oder Natriumhydrogencarbonat ist.

## Claims

1. 2-Butyl-5-(methanesulphonamido)benzofuran.

2. Method for preparing 2-butyl-5-(methanesulphonamido)benzofuran, **characterized in that** 5-amino-2-butylbenzofuran is reacted with methanesulphonyl chloride or methansulphonic anhydride, the reaction taking place in the presence of an acid acceptor, giving the desired compound.

3. Method for preparing dronedarone (2-butyl-3-(4-[3-(dibutylamino)propoxy]benzoyl)-5-(méthanesulphonamido)benzofuran), **characterized in that**, in an organic phase consisting of one or more solvents chosen from halogenated or nonhalogenated hydrocarbons, 2-butyl-5-(methanesulphonamido)benzofuran is reacted with 4-[3-(dibutylamino)propoxy]benzoyl chloride hydrochloride, this being in the presence of a maximum of 5 molar equivalents of a Lewis acid as catalyst, and then hydrolysis is carried out so as to form dronedarone hydrochloride which is recovered in the organic phase, which is isolated and which is treated with a basic agent, to give the desired compound.

4. Method according to Claim 3, **characterized in that** 2-butyl-5-(methanesulphonamido)benzofuran is reacted with 4-[3-(dibutylamino)propoxy]benzoyl chloride hydrochloride in the presence of a maximum of 4 molar equivalents of the Lewis acid.

5. Method according to Claim 3 or 4, **characterized in that** 2-butyl-5-(methanesulphonamido)benzofuran is reacted with 4-[3-(dibutylamino)propoxy]benzoyl chloride hydrochloride in the presence of a maximum of 3 molar equivalents of the Lewis acid.

6. Method for preparing dronedarone, **characterized in that**, in an organic phase consisting of one or more solvents chosen from halogenated or nonhalogenated hydrocarbons, 2-butyl-5-(methanesulphonamido)benzofuran is reacted with 4-[3-(dibutylamino)propoxy]benzoyl chloride hydrochloride, this being in the presence of a maximum of 5 molar equivalents of a Lewis acid as catalyst, and then hydrolysis is carried out in order to obtain transiently, and without isolation, dronedarone hydrochloride which is recovered in the organic phase and which is treated with a basic agent, giving the desired compound.

7. Method according to Claim 6, **characterized in that** 2-butyl-5-(methanesulphonamido)benzofuran is reacted with 4-[3-(dibutylamino)propoxy]benzoyl chloride hydrochloride in the presence of a maximum of 4 molar equivalents of the Lewis acid.

8. Method according to Claim 6 or 7, **characterized in that** 2-butyl-5-(methanesulphonamido)benzofuran is reacted with 4-[3-(dibutylamino)propoxy]benzoyl chloride hydrochloride in the presence of a maximum of 3 molar equivalents of the Lewis acid.

9. Method for preparing dronedarone, **characterized in that**:
a) 2-butyl-5-nitrobenzofuran is hydrogenated in the presence of an appropriate catalyst, to form 5-amino-2-butylbenzofuran,
b) the compound thus obtained is reacted with methanesulphonyl chloride or methanesulphonic anhydride, the reaction taking place in the presence of an acid acceptor, to form 2-butyl-5-(methanesulphonamido)benzofuran,
c) the methanesulphonamido derivative thus obtained is reacted, in an organic phase consisting of one or more solvents chosen from halogenated or nonhalogenated hydrocarbons, with 4-[3-(dibutylamino)propoxy]benzoyl chloride hydrochloride, this being in the presence of a maximum of 5 molar equivalents of a Lewis acid as catalyst, and then hydrolysis is carried out in order to obtain transiently, and without isolation, dronedarone hydrochloride which is recovered in the organic phase and which is treated with a basic agent, giving the desired compound.

10. Method according to Claim 9, **characterized in that** in step c), the methanesulphonamido derivative thus obtained is reacted with 4-[3-(dibutylamino)propoxy]benzoyl chloride hydrochloride in the presence of a maximum of 4 molar equivalents of the Lewis acid.

11. Method according to Claim 9 or 10, **characterized in that** in step c), the methanesulphonamido derivative thus obtained is reacted with 4-[3-(dibutylamino)propoxy]benzoyl chloride hydrochloride in the presence of a maximum of 3 molar equivalents of the Lewis acid.

12. Method according to one of Claims 3 to 11, **characterized in that** the organic phase consists of one or more solvents chosen from halogenated hydrocarbons of the aliphatic, alicyclic or aromatic type.

13. Method according to one of Claims 3 to 12, **characterized in that** the Lewis acid is chosen from the group consisting of aluminium chloride, zinc chloride, boron trifluoride, stannic chloride, titanium tetrachloride, ferric chloride and mixtures thereof.

14. Method according to one of Claims 3 to 13, **characterized in that** the Lewis acid is ferric chloride.

15. Method according to one of Claims 3, 6, 9, 13 and 14, **characterized in that** the Lewis acid is used in an amount of 2 to 5 molar equivalents.

16. Method according to one of Claims 3 to 11, 13 and 14, **characterized in that** the Lewis acid is used in an amount of 2 to 3 molar equivalents.

17. Method according to one of Claims 3 to 16, **characterized in that** the 4-[3-(dibutylamino)-propoxy]benzoyl chloride hydrochloride is used at concentrations of the order of 1 to 1.3 molar equivalents.

18. Method according to one of Claims 3 to 17, **characterized in that** the basic agent is sodium hydroxide or sodium hydrogen carbonate.
